Europäisches Patentamt

European Patent Office

Office européen des brevets

(19)

(11) Veröffentlichungsnummer: **0 014 977**

**A1**

(12) # EUROPÄISCHE PATENTANMELDUNG

(21) Anmeldenummer: 80100794.9

(22) Anmeldetag: 16.02.80

(51) Int. Cl.³: **A 61 B 5/02**
**G 05 D 16/06**

(30) Priorität: 21.02.79 DE 2906666

(43) Veröffentlichungstag der Anmeldung:
03.09.80 Patentblatt 80/18

(84) Benannte Vertragsstaaten:
AT BE CH DE FR GB IT LU NL SE

(71) Anmelder: CLINICON INTERNATIONAL GMBH
Sandhofer Strasse 176
D-6800 Mannheim 31(DE)

(72) Erfinder: Schüssler, Rudolf
Oberlacherstrasse 20
D-6840 Lampertheim(DE)

(72) Erfinder: van Rijckevorsel, Rainer
Freiburger Strasse 3
D-6831 Brühl(DE)

(74) Vertreter: Daum, Martin, Dr. et al,
Boehringer Mannheim GmbH. Sandhoferstrasse 116
D-6800 Mannheim 31(DE)

(54) Steuervorrichtung für die Drucksenkung in einem Blutdruckmessgerät.

(57) Die Erfindung betrifft eine Steuervorrichtung für die Drucksenkung des Druckmediums in einem Blutdruck-Meßgerät. In einem Gehäuse (1) ist ein Drosselventil (9) angeordnet, dessen Auslassquerschnitt mit einem stellbaren Ventil-Schliessteil (10) steuerbar ist. Eine gute Annäherung an einen zeitkonstanten Druckabfall während der Drucksenkung wird dabei dadurch erreicht, daß ein dem Messdruck des Druckmediums über einen Messwertkanal (41) ausgesetztes, druck-wegabhängiges Stellglied (24) für den Ventil-Schliessteil (10) vorgesehen ist.

Fig. 1

EP 0 014 977 A1

CLINICON INTERNATIONAL GMBH                    2268

Steuervorrichtung für die Drucksenkung in einem Blutdruck-
Meßgerät

Die Erfindung betrifft eine Steuervorrichtung für die Drucksenkung des Druckmediums in einem Blutdruck-Meßgerät, mit
einem in einem Gehäuse angeordneten Drosselventil, dessen
Auslaßquerschnitt zur Annäherung an einen zeitkonstanten
Druckabfall während der Drucksenkung mit einem stellbaren
Ventil-Schließteil steuerbar ist.

Bei bekannten Blutdruck-Meßgeräten dieser Art wird der Ventil-Schließteil des Drosselventiles durch Drehen einer Schraube von Hand betätigt und dadurch eine Durchlaßöffnung für das
Druckmedium mehr oder weniger geöffnet bzw. geschlossen. Zur
Erzielung einer genauen Messung soll die zeitliche Änderung
des Druckes möglichst konstant bleiben; da die Druckabnahme
auch eine Funktion des Volumens des unter Druck stehenden
Druckmediums ist, ergibt sich bei der bekannten Ausbildung
der Nachteil, daß mit sinkendem Druck das Drosselventil ständig nachreguliert werden muß, wenn man eine zeitlich gleichmäßige Sinkgeschwindigkeit des Druckes haben will. Dieses Nachregulieren ist stark von der Geschicklichkeit des jeweiligen
Benutzers abhängig.

- 2 -

Es ist auch eine Steuervorrichtung zum Steuern der Ablaßmenge des Druckmediums aus einem Blutdruck-Meßgerät bekannt (DE-AS 22 51 308), welches für den Ventil-Schließteil des Drosselventiles einen einstellbaren Anschlag aufweist, durch welchen der Grad der Öffnung des mit einem Druckknopf zu betätigenden Drosselventiles genau festgelegt ist und zur Anpassung an die Geschicklichkeit des jeweiligen Benutzers durch Verstellen des Anschlages verändert werden kann. Bei dieser Steuervorrichtung ist jedoch eine Regelung des Auslaßquerschnittes des Drosselventiles zur Annäherung an einen zeitkonstanten Druckabfall nicht vorgesehen.

Der Erfindung liegt die Aufgabe zugrunde, eine Steuervorrichtung für die Drucksenkung des Druckmediums in einem Blutdruck-Meßgerät zu schaffen, welche während des Entweichens des Druckmediums aus der am Arm des Patienten anzuordnenden Druckmanschette eine selbsttätige Veränderung des Auslaßquerschnittes des Drosselventiles derart bewirkt, daß die Druckabnahme je Zeiteinheit annähernd konstant bleibt.

Zur Lösung dieser Aufgabe ist eine Steuervorrichtung der eingangs genannten Art erfindungsgemäß gekennzeichnet durch ein dem Meßdruck des Druckmediums über einen Meßwertkanal ausgesetztes, druck-wegabhängiges Stellglied für den Ventil-Schließteil. Dadurch wird das Drosselventil in Abhängigkeit von dem nachlassenden Druck während des Entweichens des Druckmediums mehr und mehr geöffnet, wobei die relevanten Regelgrößen zweckmäßig so gewählt sind, daß sich der Ausgangsdruck von 0,25 bis 0,30 Bar während einer Dauer von etwa 60 bis 70 Sekunden konstant auf ca. 0,05 Bar abbaut. Zur Beeinflussung der Steuervorrichtung wird unmittelbar das ohnehin unter Druck zur Verfügung stehende Druckmedium des Blutdruck-Meßgerätes verwendet, so daß keine Fremdenergie erforderlich ist.

- 3 -

Zweckmäßig ist das Stellglied zur Schließ-Stellung des Drosselventiles vom Druckmedium beaufschlagt und in Öffnungs-Bewegungsrichtung des Ventil-Schließteiles mit einer vorgespannten
Rückstellfeder belastet, so daß das Ventil-Schließteil mit
dem Stellglied über einen einfachen Stelltrieb verbunden werden
kann.

Ein besonders feinfühliges Ansprechen des Stellgliedes auf
die Druckänderungen ist gewährleistet, wenn das Stellglied
als Membran ausgebildet und vorzugsweise über eine Ventilspindel im wesentlichen starr mit dem als Ventilschieber ausgebildeten Ventil-Schließteil verbunden ist. Dadurch sind zwischen dem Stellglied und dem Ventil-Schließteil keine beweglichen Teile erforderlich, so daß die Bewegungen des Stellgliedes sehr genau auf den Ventil-Schließteil übertragen werden.

Eine weitere Vereinfachung im Aufbau der Steuervorrichtung
wird erreicht, wenn der mit dem Meßwertkanal leitungsverbundene
Druckraum für das Stellglied über einen Ventilkanal unmittelbar mit dem Drosselventil leitungsverbunden ist, so daß sich
kurze Strömungswege und eine genaue Steuerung dadurch ergeben,
daß die Blutdruck-Manschette, der Druckraum für das Stellglied und das Drosselventil in Serie aneinander angeschlossen
sind.

Bei einer besonders einfachen Ausführungsform ist der Ventil-
Schließteil in einer Gehäusebohrung verschiebbar gelagert,
die im Bereich des Stellweges des Ventil-Schließteiles eine
im Bohrungsmantel liegende Drosselöffnung aufweist, die das
innere Ende eines, insbesondere radial zur Gehäusebohrung
liegenden Ausströmkanales bildet.

Durch entsprechende Formgebung der Drosselöffnung kann der jeder Stellung des Ventil-Schließteiles und damit dem jeweiligen Druck des Druckmediums entsprechende Auslaßquerschnitt des Drosselventiles genau festgelegt werden. Zweckmäßig nimmt der Durchlaßquerschnitt des Drosselventiles mit zunehmender Drosselöffnung progressiv zu. Dies ist z.B. in einfacher Weise dadurch zu erreichen, daß die Drosselöffnung in Öffnungs-Bewegungsrichtung des Ventil-Schließteiles in ihrer Weite zunimmt, wobei sich als besonders vorteilhaft erwiesen hat, wenn die Drosselöffnung in dieser Richtung durch spitzwinklig gleichschenklige, dreieckförmige Begrenzung erweitert ist.

Zur noch genaueren Annäherung an einen zeitkonstanten Druckabfall hat es sich als vorteilhaft erwiesen, wenn der Ventil-Schließteil einen gegenüber der Gehäusebohrung verringerten Querschnitt, insbesondere einen kleineren Durchmesser, aufweist und vorzugsweise an seinem in Öffnungs-Bewegungsrichtung hinteren Ende einen konisch oder ähnlich verjüngten Abschnitt aufweist, so daß insbesondere gegen Ende der Drucksenkung, bei welchem nur noch sehr niedrige, schwer zu beherrschende Drücke vorliegen, die Zeitkonstanz bzw. Linearität des Druckabfalls aufrechterhalten werden kann.

Nach einem weiteren Merkmal der Erfindung ist ein Ende des Ventil-Schließteiles, insbesondere dessen in Öffnungs-Bewegungsrichtung hinteres Ende über die in der Gehäusebohrung liegende Ventilspindel mit dem benachbart zum zugehörigen Ende der Gehäusebohrung liegenden Stellglied verbunden, so daß eine baulich kompakte Ausbildung gewährleistet ist. Zweckmäßig begrenzt der zwischen dem Ventil-Schließteil und dem Stellglied liegende Abschnitt der Ventilspindel mit der Gehäusebohrung den Ventilkanal, wobei vorzugsweise ein unmittelbar an den Ventil-Schließteil anschließender Teil dieses Spindelabschnittes im Querschnitt annähernd kreisabschnittförmig und an der Bohrungswandung geführt ist, so daß trotz Schaffung des Durchflußquerschnittes für das Druckmedium eine sehr genaue Führung des Ventil-Schließteiles gewährleistet ist. Zur weiteren Verbesserung dieser Führung ist an dem in Öffnungs-Bewegungsrichtung vorderen Ende des Ventil-Schließteiles ebenfalls ein Spindelabschnitt vorgesehen, welcher in der Gehäusebohrung geführt ist.

Bei einer baulich einfachen Ausführungsform liegt der Meßwertkanal etwa parallel zur Gehäusebohrung, wobei er mit einem Ende in den dem Stellglied zugehörigen, insbesondere gegenüber der Gehäusebohrung erweiterten, Druckraum mündet und mit dem anderen Ende in einen beispielsweise radialen Anschlußkanal für den Anschluß der Blutdruck-Manschette übergeht. Ferner ist es vorteilhaft, wenn das von dem Stellglied abgekehrte Ende der Gehäusebohrung in einen Anschluß für eine Pumpe für das Druckmedium, beispielsweise für einen Pumpballon, übergeht und wenn vorzugsweise in diesem Ende der Gehäusebohrung ein zum Anschluß hin schließendes Rückschlagventil angeordnet ist, so daß dieses Rückschlagventil und der Ventil-Schließteil in derselben, als Durchgangsbohrung einfach herzustellenden Gehäusebohrung angeordnet werden können.

Diese Ausbildung hat ferner den Vorteil, daß der Anschlußkanal in einfacher Weise zwischen dem Ventil-Schließteil bzw. dem zugehörigen Spindelabschnitt und dem Rückschlagventil in die Gehäusebohrung münden kann und dadurch mit dem Pumpballon auf einfache Weise leitungsverbunden ist.

Eine besonders vorteilhafte Weiterbildung des Erfindungsgegenstandes besteht darin, daß im Ablaßweg des Druckmediums, insbesondere in Strömungsrichtung nach dem Drosselventil, ein

- 6 -

handbetätigbares Sperrventil mit einem gegenüber dem größten Druchlaßquerschnitt des Drosselventiles größeren Durchlaßquerschnitt angeordnet ist. Dadurch kann der Beginn der Drucksenkung durch Öffnen dieses Sperrventiles genau festgelegt werden, wobei der Ventil-Schließteil des Drosselventiles bereits vor dem Öffnen des Sperrventiles in der beschriebenen Weise derart angesteuert ist, daß er sich in einer Stellung befindet, bei welcher der Durchlaßquerschnitt des Drosselventiles verhältnismäßig klein ist.

Das Sperrventil kann in einfacher Weise und raumsparend in dem Ausströmkanal liegen und weist vorzugsweise einen als federbelasteten Druckstößel ausgebildeten Schließteil auf, so daß es einfach zu betätigen ist.

Eine andere vorteilhafte Weiterbildung des Erfindungsgegenstandes besteht ferner darin, daß in Parallelschaltung zum Drosselventil bzw. zum Sperrventil ein an den Anschlußkanal für die Blutdruck-Manschette angeschlossener Zweigkanal vorgesehen ist, in welchem ein handbetätigbares Schnellablaß-Ventil angeordnet ist, dessen Durchlaßquerschnitt gegenüber dem größten Durchlaßquerschnitt des Drosselventiles ebenfalls wesentlich größer ist. Mit diesem Schnellablaß-Ventil kann, wenn erwünscht, das Druckmedium aus der Blutdruck-Manschette unter Umgehung des Drosselventiles auch schlagartig in kürzester Zeit abgelassen werden.

Das Sperrventil und das Schnellablaß-Ventil können in einfacher Weise gleich ausgebildet und/oder raumsparend hintereinander an dem Gehäuse angeordnet sein.

Zur Vereinfachung der Bedienung des Blutdruck-Meßgerätes ist zweckmäßig bei den handbetätigbaren Ventilen eine gemeinsame Handhabe, insbesondere ein Schiebeknopf, zugeordnet, der

- 7 -

Keilflächen für die Anlage an Druckflächen der Ventil-Stößel der beiden Ventile aufweist.

Nach einem weiteren Vorschlag gemäß der Erfindung ist die Membran mit einem äußeren Ringflansch zwischen einer Schulterfläche des Gehäuses und einem Enddeckel des Gehäuses eingespannt und im Bereich ihres Zentrums mit dem Ventil-Schließteil des Drosselventiles verbunden, so daß der Aufbau der Steuervorrichtung weiter vereinfacht wird.

Um die Ausgangslage des Ventil-Schließteiles zu Beginn der Drucksenkung genau festlegen zu können, ist der Stellweg des Stellgliedes mit einem einstellbar gelagerten Anschlag begrenzt, der vorzugsweise durch eine in der Rückstellfeder liegende Gewindespindel gebildet ist, die im Enddeckel des Gehäuses gelagert ist.

Die Erfindung wird im folgenden mit weiteren Einzelheiten erläutert; die Zeichnungen geben zwei Ausführungsbeispiele annähernd maßstabgerecht wieder. Es sind dargestellt in

> Fig. 1 eine erfindungsgemäße Steuervorrichtung im Axialschnitt,
>
> Fig. 2 das Gehäuse der Steuervorrichtung gemäß Fig. 1 in Draufsicht,
>
> Fig. 3 ein Schnitt nach der Linie III-III in Fig. 1,
>
> Fig. 4 der Ventilschieber gemäß Fig. 1 in Ansicht von oben,
>
> Fig. 5 eine Draufsicht auf die Steuervorrichtung gemäß Fig. 1,

Fig 6 eine weitere Ausführungsform einer Steuervorrichtung im Axialschnitt,

Fig 7 ein Ausschnitt des Ventilschiebers gemäß Fig. 6
in Ansicht von oben.

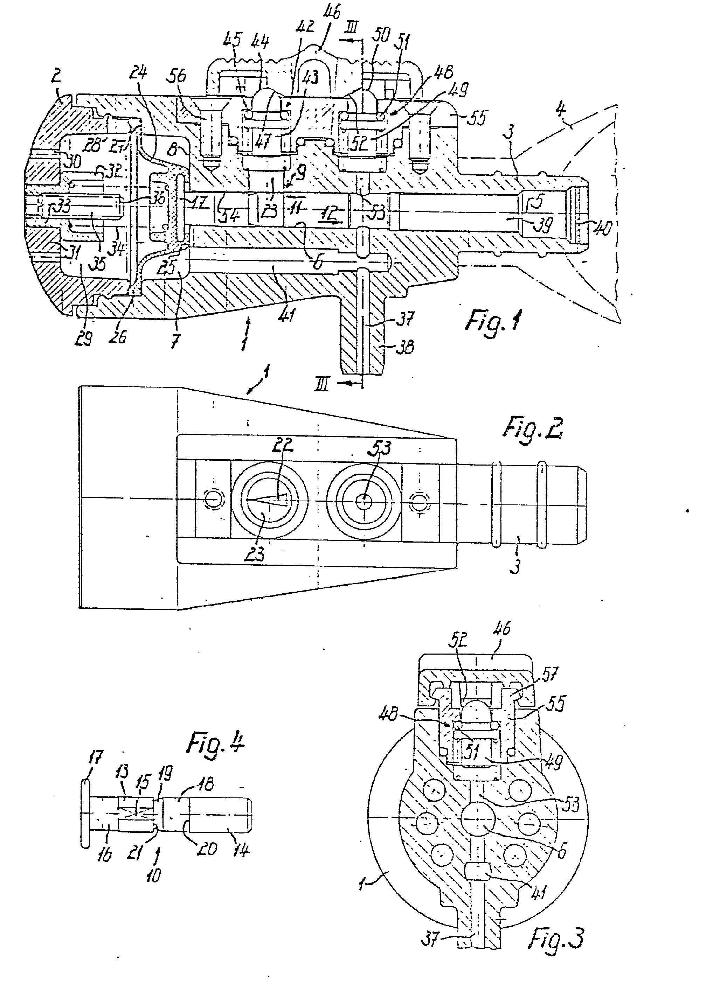

Wie die Fig. 1 bis 5 zeigen, weist eine erfindungsgemäße
Steuervorrichtung ein längliches, zu einer Axialebene im
wesentlichen symmetrisches Gehäuse 1 auf, das an einem Ende
mit einem lösbaren Enddeckel 2 verschlossen ist und am anderen Ende einen Anschluß 3 für einen strichpunktiert angedeuteten Pumpballon 4 aufweist.

In der Mittelachse 5 des Gehäuses 1 ist eine durchgehende
Gehäusebohrung 6 von wenigen, beispielsweise etwa 4 mm Durchmesser und hoher Oberflächengüte sowie Genauigkeit vorgesehen.
Die Bohrung geht an dem dem Enddeckel 2 zugekehrten Ende innerhalb des Gehäuses 1 in einen im Durchmesser erweiterten, in
der Mittelachse 5 liegenden, annähernd zylindrisch begrenzten
Druckraum 7 über, der an seinem inneren Ende von einer ebenen,
zur Mittelachse 5 rechtwinkligen Stirnfläche 8 begrenzt ist.

In der Gehäusebohrung 6 ist der Ventil-Schließteil 10 eines
Drosselventiles 9 in entgegengesetzten Richtungen, Pfeil 11
und Pfeil 12, verschiebbar gelagert. Der an einer Ventilspindel vorgesehene Ventil-Schließteil 10 gemäß Fig. 4 weist an
beiden Enden jeweils einen Spindelabschnitt 13 bzw. 14 auf.
Der in Öffnungs-Bewegungsrichtung Pfeil 12 hintere Spindelabschnitt 13 hat zylindrische Grundform mit einem Durchmesser,
welcher an den der Gehäusebohrung 6 so angepaßt ist, daß der
Spindelabschnitt 13 leichtgängig und genau in der Gehäusebohrung 6 geführt ist. An einer Stelle seines Umfanges weist
dieser Spindelabschnitt 13 eine über seine Länge durchgehende
Abflachung 15 auf, so daß diese Abflachung 15 mit der Gehäuse- bohrung 6 einen Kanal begrenzt. Der andere, ebenfalls zylindrische und geringfügig längere Spindelabschnitt 14 ist annähernd über seine ganze Länge und über seinen ganzen Umfang derart an den Durchmesser der Gehäusebohrung 6 angepasst, dass er an dieser leichtgängig und genau geführt ist. An das vom Ventil-Schliessteil 10 abgekehrte Ende des Spindelabschnittes 13 schliesst sich ein weiterer, etwa gleich langer Spindelabschnitt 16 an, der gegenüber der Gehäusebohrung 6 kleineren Durchmesser, beispielsweise einen Durchmesser von etwa 3,6 mm hat und in welchen die Abflachung 15 im Bereich der Mitte ihrer Breite tangential übergeht. Am freien Ende dieses Spindelabschnittes 16 ist ein scheibenförmiges, im Durchmesser erweiterte und einen abgerundeten Umfang aufweisendes Formschlussglied 17 vorgesehen. Der Ventil-Schliessteil 10 weist im Anschluss an den Spindelabschnitt 14 einen zylindrischen, im Durchmesser geringfügig, nämlich z.B. nur um 8/100 mm gegenüber der Gehäusebohrung 6 reduzierten Abschnitt 18 auf, welcher in einem zum Spindelabschnitt 13 annähernd stetig im Durchmesser reduzierten, z.B. kegelstumpfförmigen Abschnitt 19 übergeht, der wesentlich kürzer als der Abschnitt 18, nämlich z.B. nur 1 mm lang ist. Der Abschnitt 18 geht in den Spindelabschnitt 14 über eine Ringschulter 20 über. Entsprechend geht der Abschnitt 19, der in seinem an den Abschnitt 18 anschliessenden Bereich gleichen Aussendurchmesser wie der Abschnitt 18 hat, in den Spindelabschnitt 13 über eine Ringschulter 21 über.

Im Bereich des Bewegungsweges des Ventil-Schliessteiles ist in der Wandung der Gehäusebohrung 6 eine Drosselöffnung 22 vorgesehen, deren Erstreckung in Längsrichtung der Gehäusebohrung 6 gleich dem Abstand zwischen den Ringschultern 20, 21 des Ventil-Schliessteiles 10 ist, wobei diese Erstreckung gleich dem Durchmesser der Gehäusebohrung 6 ist. Die Drosselöffnung 22 ist symmetrisch zu der durch die Mitte ihrer Breite gehenden Axialebene der Mittelachse 5 ausgebildet und in Ansicht unter einem spitzen Winkel von 20° gleichschenklig dreieckförmig begrenzt, wobei ihre Spitze zum Druckraum 7 weist. Die Drosselöffnung 22 bildet das innere Ende eines radial zur Mittelachse 5 liegenden Ausströmkanales 23, dessen Mittelachse in der genannten Axialebene der Drosselöffnung 22 liegt und welcher aus dem Gehäuse 1 herausführt.

Das Formschlußglied 17 der Ventilspindel liegt in dem Druckraum 7 und ist durch federndes Einspringen in der Nabe 25 eines als gummielastische Membran ausgebildeten Stellgliedes 24 befestigt. Dieses Stellglied 24 weist um die Nabe 25 einen ringförmigen, elastischen Membranteil auf, der am Außenumfang in einen verdickten Ringflansch 26 übergeht. Der Ringflansch 26 ist zwischen einem inneren, im Durchmesser reduzierten hülsenförmigen Ende des Enddeckels 2 und einer Ringschulter 27 des Gehäuses 1 eingespannt, welche das zugehörige Ende des Druckraumes 7 umgibt. Der Enddeckel 2 weist an seinem in das Gehäuse 1 ragenden Ende einen Ringwulst 28 auf, welcher in eine entsprechende Ringnut des Gehäuses 1 eingreift, so daß der Enddeckel 2 lediglich durch Eindrücken in das Gehäuse 1 befestigt werden kann. Der im wesentlichen vom Enddeckel 2 begrenzte, auf der vom Druckraum 7 abgekehrten Seite des Stellgliedes 24 liegende Raum 29 ist durch Bohrungen 30 belüftet, welche um die Mittelachse 5 in der Stirnwand des Enddeckels 2 angeordnet sind und ins Freie führen.

In der Stirnwand 31 des in der Mittelachse 5 liegenden Enddeckels 2 ist ferner ein hülsenförmiges Widerlager 32 mit einem im Durchmesser reduzierten Außengewinde-Abschnitt in einer die Stirnwand 31 durchsetzenden Gewindebohrung befestigt. In das an der Innenseite der Stirnwand 31 des Enddeckels 2 in der Mittelachse 5 liegende Widerlager 32 greift eine als Schraubendruckfeder ausgebildete, vorgespannte Rückstellfeder 34 ein, die mit dem zugehörigen Ende an einer Ringschulter des Widerlagers 32 anliegt und deren zugehöriges Ende durch das Widerlager 32 zentriert ist. Das andere Ende der Rückstellfeder 34 liegt zentriert an der zugehörigen Stirnseite der Nabe 25 des Stellgliedes 24 an, derart, daß die Nabe 25 zur Stirnwand 8 des Druckraumes 7 federbelastet ist und in der entsprechenden Endstellung mit ihrer das Formschlußglied 17 übergreifenden ringförmigen Stirnfläche an der Stirnfläche 8 anliegt. In einem in der Mittelachse 5 liegenden Innengewinde des Außengewinde-Abschnittes 33 des Widerlagers 32 ist eine Stellschraube 35 in Bewegungsrichtung des Ventil-Schließteiles 10 verstellbar gelagert, deren der Nabe 25 des Stellgliedes 24 zugekehrtes Ende einen Anschlag für die ihm zugekehrte Stirnfläche der Nabe 25 bildet. Die teilweise innerhalb der Rückstellfeder 34 liegende Stellschraube 35 ist von der äußeren Stirnseite des Enddeckels 2 her jederzeit zur Einstellung zugänglich. Sie begrenzt die andere Endstellung des Stellgliedes 24 und damit des Ventil-Schließteiles 10.

In der Axialebene der Mittelachse 5, in welcher die Drossel-Öffnung 22 liegt, jedoch auf der anderen Seite dieser Mittelachse 5, ist in dem Gehäuse 1 ein im Durchmesser gegenüber der Gehäusebohrung 6 wesentlich kleinerer Anschlußkanal 37 vorgesehen, der teilweise durch einen über das Gehäuse radial zur Mittelachse 5 vorstehenden Anschlußstutzen 38 begrenzt ist. Auf diesen Anschlußstutzen 38 wird ein nicht näher dargestellter Schlauch zur Verbindung der Steuervorrichtung mit der Blutdurck-Manschette aufgesteckt. Der Anschlußkanal 37 mündet benachbart zu dem in Öffnungs-Bewegungsrichtung Pfeil 12 vorderen Ende des Ventilschiebers in die Gehäusebohrung 6. Auf der vom Ventilschieber abgekehrten Seite des Anschlußkanales 37 ist in einem im Durchmesser geringfügig erweiterten Abschnitt der Gehäusebohrung 6 ein Rückschlagventil 39 angeordnet, welches teilweise bis in den stutzenförmigen Anschluß 3 reicht, auf welchen der Pumpballon 4 mit einem entsprechend hülsenförmigen Abschnitt aufgesteckt ist. Zwischen dem Innenraum des Pumpballons 4 und dem Rückschlagventil 39 ist in der Gehäusebohrung noch ein Luftfilter 40 angeordnet. Das Rückschlagventil 39 schließt bei Überdruck in der Gehäusebohrung 6 bzw. im Anschlußkanal 37; der Pumpballon 4 weist in einer nicht näher dargestellten, ins Freie führenden Ansaugöffnung ebenfalls ein Rückschlagventil auf, welches bei Überdruck im Pumpballon 4 schließt. Durch Zusammendrücken und Freigeben des Pumpballons 4 wird durch den Luftfilter 40, das Rückschlagventil 39, den Anschlußkanal 37 und den genannten Schlauch Luft in die Blutdruck-Manschette gepumpt.

In der Axialebene der Mittelachse 5, in welcher die Drosselöffnung 22 vorgesehen ist, jedoch auf der dieser gegenüberliegenden Seite der Mittelachse 5, ist ferner benachbart zur Gehäusebohrung 6 im Gehäuse 1 ein zur Gehäusebohrung 6 paralleler Meßwertkanal 41 vorgesehen, dessen eines, die Stirnwand 8 benachbart zur Umfangsfläche des Druckraumes 7 durchsetzendes Ende in den Druckraum 7 mündet, während das andere, sacklochartig geschlossene Ende den Anschlußkanal 37 durchdringt, so daß der Druckraum 7 stets von dem in der Blutdruck-Manschette herrschenden Druck beaufschlagt wird.

Im Ausströmkanal 23 ist in einem gegenüber der Drosselöffnung 22 erweiterten, benachbart zur Außenseite des Gehäuses 1 liegenden Abschnitt ein Sperrventil 42 angeordnet, das einen achssymmetrischen Ventilstößel 43 aufweist, welcher am äußeren Ende mit einer kugelkalottenförmigen Druckfläche 44 versehen ist. Der Ventilstößel 43 weist zum dichten Absperren des Ausströmkanales 23 einen O-Ring 45 auf, dem im Gehäuse 1 eine entsprechende, ringförmige Schließfläche zugeordnet ist, gegen welche der O-Ring 45 bei geschlossenem Sperrventil 42 durch die Kraft einer vorgespannten, das innere Ende des Ventilstößels 43 umgebenden Feder gedrückt wird. Durch Hineindrücken des Ventilstößels 43 in den Ausströmkanal 23 wird das Sperrventil 42 entgegen der Kraft der Feder geöffnet. An der zugehörigen Seite des Gehäuses 1 ist an dessen Außenseite eine Handhabe 46 in Form eines Schiebeknopfes in Längsrichtung des Gehäuses 1 verschiebbar gelagert. Die Handhabe 46, die das vorstehende Ende des Ventilstößels 43 überdeckt, weist an ihrer Unterseite eine Keilfläche 47 auf, welche beim Verschieben der Handhabe 46 in Richtung zum Enddeckel 2 auf die Druckfläche 44 aufläuft und dadurch den Ventilstößel 43 in seine Öffnungsstellung überführt und in dieser Öffnungsstellung festhält, bis die Handhabe 46 wieder in ihre Ausgangslage zurückgeschoben wird.

Benachbart zum Sperrventil 42 und in derselben Axialebene der Mittelachse 5 wie dieses ist ferner ein Schnellablaß-Ventil 48 im Gehäuse 1 angeordnet, das auf der vom Enddeckel 2 abgekehrten Seite des Sperrventiles 42 liegt und gleich wie dieses ausgebildet ist. Demnach weist dieses Ventil 48 ebenfalls einen gegen die Kraft einer Feder bewegbaren Ventilstößel 49, eine vorstehende Druckfläche 50 und als Dichtung einen O-Ring 51 auf, welcher in Schließstellung dieses Ventiles 48 an einer entsprechenden Ringfläche anliegt. Dem ebenfalls von der Handhabe 46 überdeckten Ventilstößel 49 ist eine weitere, entgegengesetzt zur Keilfläche 47 ansteigende Keilfläche 52 zugeordnet, mit welcher der Ventilstößel 49 entgegen der Kraft der Feder in Öffnungsstellung überführt wird, wenn die Handhabe 46 auf ihrer Ausgangsstellung gemäß Fig. 1 in Richtung zum Pumpballon 4 verschoben wird; die Handhabe 46 hält dabei das Schnellablaß-Ventil 48 so lange offen, bis sie wieder in ihre Ausgangsstellung zurückgeschoben wird, in welcher die beiden Keilflächen 47, 52 zwischen den beiden Druckflächen 44, 50 liegt. Mit der

- 14 -

Handhabe 46 kann demnach wahlweise immer nur eines der beiden Ventile geöffnet oder können gleichzeitig beide Ventile geschlossen werden.

Das Schnellablaß-Ventil 48 ist in einem im Durchmesser erweiterten Abschnitt eines Zweigkanales 53 angeordnet, welcher mit dem Anschlußkanal 37 dadurch leitungsverbunden ist, daß dieser, im Gehäuse 1 liegende Zweigkanal 53 zwischen der Ventilspindel und dem Rückschlagventil 39 in die Gehäusebohrung 6 mündet. Der Zweigkanal 53 fluchtet dabei mit dem Anschlußkanal 37.

Zum Betrieb des Blutdruck-Meßgerätes wird die am Arm des Patienten angelegte Blutdruck-Manschette durch Pumpen mit dem Pumpballon 4 in der beschriebenen Weise mit Luft gefüllt, wobei sowohl das Sperrventil 42 als auch das Schnellablaß-Ventil 48 geschlossen sind. Mit der Erhöhung des Druckes in der Blutdruck-Manschette wird auch im Druckraum 7 ein entsprechender Druck aufgebaut, welcher die Nabe 25 des Stellgliedes 24 gegen die Kraft der Rückstellfeder 34 zum Anschlag 36 hin bewegt und dadurch den Ventil-Schließteil 10 entsprechend gegenüber der Drosselöffnung 22 verstellt. Der Stellweg des Ventil-Schließteiles 10 entspricht dabei der Erstreckung der Drosselöffnung 22 in Bewegungsrichtung des Ventil-Schließteiles 10. In der zugehörigen, anschlagbegrenzten Endstellung des Stellgliedes 24 liegt die Ringschulter 20 des Ventil-Schließteiles 10 nahezu am spitzen Ende der Drosselöffnung 22, so daß nur ein äußerst kleiner Durchlaßquerschnitt des Drosselventiles 9 offen ist. Wird nunmehr durch Betätigen der Handhabe 46 das Sperrventil 42 geöffnet, so strömt die Luft aus der Blutdruck-Manschette durch den Anschlußkanal 37, den Meßwertkanal 41, den Druckraum 7 und einen Verbindungskanal 54, welcher durch die Ventilspindel und die Gehäusebohrung 6 begrenzt wird, durch den offenen Querschnitt der Drosselöffnung 22 und das geöffnete Sperrventil 42 ins Freie. Dadurch verringert sich der Druck im Druckraum 7 entsprechend dem Druck in der Blutdruck-Manschette, so daß das Stellglied 24 unter der Kraft der Rückstellfeder 34 annähernd proportional zum Druckabfall zu seiner in Fig. 1 dargestellten Ausgangslage hin bewegt wird. Entsprechend wird durch das Stellglied 24 auch der Ventil-Schließteil 10 bewegt, so daß der offene Querschnitt der Drosselöffnung 22 mit zunehmendem Stellweg vergrößert wird. Dadurch ergibt sich ein zeitkonstanter Druckabfall in der Blutdruck-Manschette. Am Ende der Stellbewegung des Ventil-Schließteiles 10 liegt die Ringschulter 20 im Bereich des breiteren Endes der Drosselöffnung 22, so daß der größtmögliche Durchlaßquerschnitt erreicht ist. Die Vorspannung der Rückstellfeder 34 kann durch Verstellen des Widerlagers 32 im Enddeckel 2 ebenfalls verändert werden.

Soll statt dem Ablassen der Druckluft aus der Blutdruck-Manschette in der zuletzt beschriebenen Weise ein beschleunigtes Ablassen erfolgen, so wird das Schnellablaß-Ventil 48 durch entsprechende Betätigung der Handhabe 46 geöffnet, so daß die Druckluft aus der Blutdruck-Manschette über den Anschlußkanal 37, die Gehäusebohrung 6, den Zweigkanal 53 und das Schnellablaß-Ventil 48 unter Umgehung des Drosselventiles 9 schnell entweicht.

Wie die Fig. 1 und 3 ferner zeigen, sind das Sperrventil 42 und das Schnellablaß-Ventil 48 in einem gesonderten Gehäusekörper 55 angeordnet, welcher in eine entsprechende Vertiefung an der zugehörigen Seite des Gehäuses 1 eingreift und mit Schrauben 56 am Gehäuse 1 befestigt ist. Dadurch lassen sich die Ventilstößel 43, 49 mit den O-Ringen 45, 51 und den zugehörigen Ventilfedern leicht von der Innenseite des Gehäusekörpers 55 her montieren. Die im Querschnitt gemäß Fig. 3 annähernd U-förmige Handhabe 46 hintergreift mit ihren Schenkeln Gleitführungen 57 des Gehäusekörpers 55, wobei die Handhabe 46 lediglich durch federndes Aufdrücken auf die Gleitführungen 57 an dem Gehäusekörper 55 befestigt ist.

- 16 -

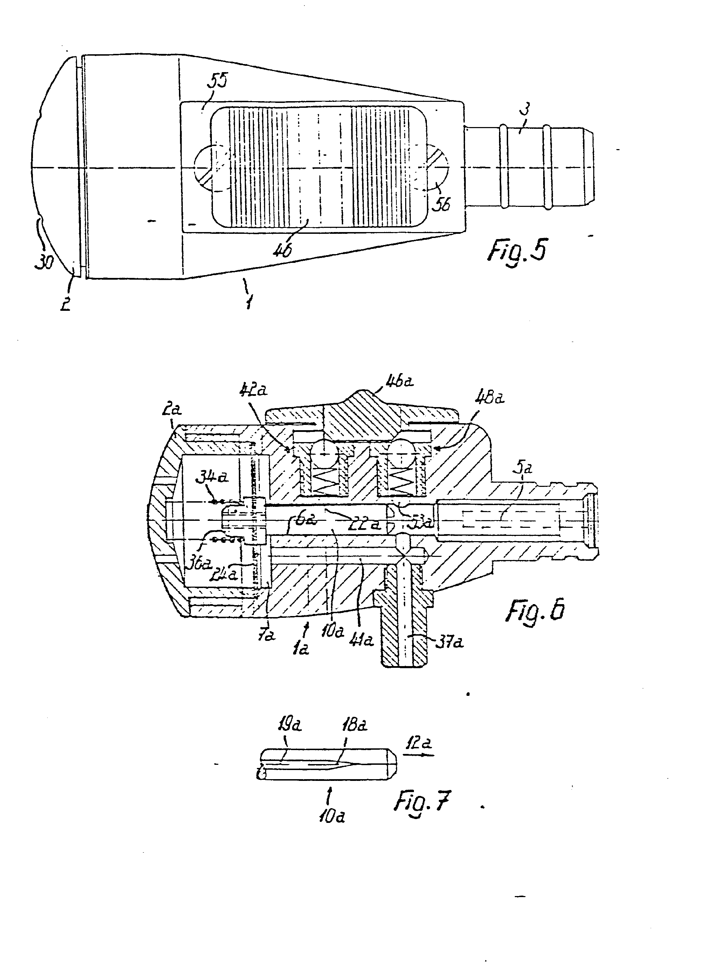

In den Fig. 6 und 7 sind für einander entsprechende Teile die gleichen Bezugzeichen wie in den Fig. 1 bis 5, jedoch mit dem Index "a" verwendet. Bei der Ausführungsform nach den Fig. 6 und 7 ist die Drosselöffnung 22a in Ansicht kreisrund und durch das innere Ende einer entsprechenden Bohrung gebildet. Der über seine Länge durchgehend gleichen Durchmesser wir die Gehäusebohrung 6a aufweisende Ventil-Schließteil 10a weist entlang einer Mantellinie am Umfang eine zur Mittelachse 5a parallele Abflachung auf, die an ihrem in Öffnungs-Bewegungsrichtung Pfeil 12a vorderen Ende in ihrem Abstand zur zylindrischen Mantelfläche des Ventil-Schließteiles 10a abnimmt und dadurch spitz ausläuft. Dadurch gibt die Abflachung 19a bei der Bewegung des Ventil-Schließteiles 10a in Pfeilrichtung 12a einen progressiv zunehmenden Durchlaßquerschnitt der Drosselöffnung 22a frei, so daß hinsichtlich des linearen Druckabfalles eine ähnliche Wirkung wie bei der Ausführungsform nach den Fig. 1 bis 6 erzielt wird. Der Ventil-Schließteil 10a ist derart verdrehgesichert in der Gehäusebohrung 6a geführt, daß die Drosselöffnung 22a stets im Bewegungsweg der Abflachung 19a liegt.

### A n s p r ü c h e

1. Steuervorrichtung für die Drucksenkung des Druckmediums in einem Blutdruck-Messgerät, mit einem in einem Gehäuse angeordneten Drosselventil, dessen Auslassquerschnitt zur Annäherung an einen zeitkonstanten Druckabfall während der Drucksenkung mit einem stellbaren Ventil-Schliessteil steuerbar ist, gekennzeichnet durch ein dem Messdruck des Druckmediums über einen Messwertkanal (41) ausgesetztes, druck-wegabhängiges Stellglied (24) für den Ventil-Schliessteil (10).

2. Vorrichtung nach Anspruch 1, dadurch gekennzeichnet, dass das Stellglied (24) zur Schliess-Stellung des Drosselventiles vom Druckmedium beaufschlagt und in Oeffnungs-Bewegungsrichtung (Pfeil 12) des Ventil-Schliessteiles (10) mit einer vorgespannten Rückstellfeder (34) belastet ist.

3. Vorrichtung nach Anspruch 1 oder 2, dadurch gekennzeichnet, dass das Stellglied als Membran (24) ausgebildet und vorzugsweise über eine Ventilspindel im wesentlichen starr mit dem als Ventilschieber ausgebildeten Ventil-Schliessteil (10) verbunden ist.

4. Vorrichtung nach einem der Ansprüche 1 bis 3, dadurch gekennzeichnet, dass der mit dem Messwertkanal (41) leitungsverbundene Druckraum (7) für das Stellglied (24) über einen Ventilkanal (54) unmittelbar mit dem Drosselventil (9) leitungsverbunden ist.

5. Vorrichtung nach einem der Ansprüche 1 bis 4, dadurch gekennzeichnet, dass der Ventil-Schliessteil (10) in einer Gehäusebohrung (6) verschiebbar gelagert ist, die im Bereich des Stellweges des Ventil-Schliessteiles eine im

Bohrungsmantel liegende Drosselöffnung (22) aufweist, die das innere Ende eines, insbesondere radial zur Gehäusebohrung (6) liegenden Ausströmkanales (23) bildet.

6. Vorrichtung nach einem der Ansprüche 1 bis 5, dadurch gekennzeichnet, dass der Durchlassquerschnitt des Drosselventiles (9) mit zunehmender Drosselöffnung progressiv zunimmt.

7. Vorrichtung nach einem der Ansprüche 1 bis 6, dadurch gekennzeichnet, dass die Drosselöffnung (22) in Oeffnungs-Bewegungsrichtung (Pfeil 12) des Ventil-Schliessteiles (10) in ihrer Weite zunimmt, insbesondere spitzwinklig gleichschenklig dreieckförmig begrenzt ist.

8. Vorrichtung nach einem der Ansprüche 5 bis 7, dadurch gekennzeichnet, dass der Ventil-Schliessteil (10) einen gegenüber der Gehäusebohrung (6) verringerten Querschnitt, insbesondere einen kleineren Durchmesser aufweist und vorzugsweise an seinem in Oeffnungs-Bewegungsrichtung (Pfeil 12) hinteren Ende einen konisch oder ähnlich verjüngten Abschnitt (19) aufweist.

9. Vorrichtung nach einem der Ansprüche 5 bis 8, dadurch gekennzeichnet, dass ein Ende des Ventil-Schliessteiles (10), insbesondere dessen in Oeffnungs-Bewegungsrichtung (Pfeil 12) hinteres Ende über die in der Gehäusebohrung (6) liegende Ventilspindel mit dem benachbart zum zugehörigen Ende der Gehäusebohrung (6) liegenden Stellglied (24) verbunden ist.

10. Vorrichtung nach Anspruch 9, dadurch gekennzeichnet, dass der zwischen dem Ventil-Schliessteil (10) und dem Stellglied (24) liegende Abschnitt (13, 16) der Ventilspindel mit der Gehäusebohrung (6) den Ventilkanal (54) begrenzt und dass vorzugsweise ein unmittelbar an den Ventil-Schliessteil (10) anschliessender Teil (13) dieses Spindelabschnittes (13, 16) im Querschnitt annähernd kreisabschnittförmig und an der Bohrungswandung geführt ist.

11. Vorrichtung nach einem der Ansprüche 5 bis 10, dadurch gekennzeichnet, dass der Messwertkanal (41) etwa parallel zur Gehäusebohrung (6) liegt, mit einem Ende in den dem Stellglied (24) zugehörigen, insbesondere gegenüber der Gehäusebohrung (6) erweiterten Druckraum (7) mündet und mit dem anderen Ende in einen beispielsweise radialen Anschlusskanal (37) für den Anschluss einer Blutdruck-Manschette übergeht.

12. Vorrichtung nach einem der Ansprüche 5 bis 11, dadurch gekennzeichnet, dass das von dem Stellglied (24) abgekehrte Ende der Gehäusebohrung (6) in einen Anschluss (3) für eine Pumpe für das Druckmedium, beispielsweise für einen Pumpballon (4) übergeht und dass vorzugsweise in diesem Ende der Gehäusebohrung (6) ein zum Anschluss (3) hin schliessendes Rückschlagventil (39) angeordnet ist und daß insbesondere der Anschlusskanal (37) zwischen dem Ventil-Schließteil (10) bzw. dem zugehörigen Spindelabschnitt (14) und dem Rückschlagventil (39) in die Gehäusebohrung (6) mündet.

13. Vorrichtung nach einem der Ansprüche 1 bis 12, dadurch gekennzeichnet, dass im Ablassweg des Druckmediums, insbesondere in Strömungsrichtung nach dem Drosselventil (9) ein handbetätigbares Sperrventil (42) mit einem gegenüber dem grössten Durchlassquerschnitt des Drosselventiles grösseren Durchlassquerschnitt angeordnet ist und daß insbesondere das Sperrventil (42) in dem Ausströmkanal (23) liegt und vorzugsweise einen als federbelasteten Druckstössel ausgebildeten Schliessteil (43) aufweist.

14. Vorrichtung nach einem der Ansprüche 1 bis 13, dadurch gekennzeichnet, dass in Parallelschaltung zum Drosselventil (9) bzw. zum Sperrventil (42) ein an den Anschlusskanal (37) für die Blutdruck-Manschette angeschlossener Zweigkanal (53) vorgesehen ist, in welchem ein handbetätigbares Schnellablass-Ventil (48) angeordnet ist.

15. Vorrichtung nach einem der Ansprüche 13 oder 14 dadurch gekennzeichnet, dass beiden handbetätigbaren Ventilen (42, 48) eine vorzugsweise gemeinsame Handhabe (46), insbesondere ein Schiebeknopf zugeordnet ist, der Keilflächen (47, 52) für die Anlage an Druckflächen (44,50) der Ventilstößel (43,49) der beiden Ventile (42,48) aufweist.

16. Vorrichtung nach einem der Ansprüche 3 bis 15, dadurch gekennzeichnet, dass die Membran (24) mit einem äusseren Ringflansch (26) zwischen einer Schulterfläche (27) des Gehäuses (1) und einem Enddeckel (2) des Gehäuses (1) eingespannt und im Bereich ihres Zentrums mit dem Ventil-Schliessteil (10) des Drosselventiles (9) verbunden ist.

17. Vorrichtung nach einem der Ansprüche 1 bis 16, dadurch gekennzeichnet, dass der Stellweg des Stellgliedes mit einem einstellbar gelagerten Anschlag (36) begrenzt ist, der vorzugsweise durch eine in der Rückstellfeder (34) liegende Gewindespindel (35) gebildet ist, die im Enddeckel (2) des Gehäuses (1) gelagert ist.

Fig. 1

Fig. 2

Fig. 4

Fig. 3

Fig.5

Fig.6

Fig.7

**Europäisches Patentamt**

**EUROPÄISCHER RECHERCHENBERICHT**

Nummer der Anmeldung

**EP 80 10 0794**

| | EINSCHLÄGIGE DOKUMENTE | | KLASSIFIKATION DER ANMELDUNG (Int.Cl.) |
|---|---|---|---|
| Kategorie | Kennzeichnung des Dokuments mit Angabe, soweit erforderlich, der maßgeblichen Teile | betrifft Anspruch | A 61 B 5/02<br>G 05 D 16/06 |
| X | US - A - 3 504 663 (W.C. EDWARDS/ SMITH KLINE & FRENCH LABORATORIES)<br> * Spalte 1, Zeile 11 - Spalte 3, Zeile 14; Abbildungen 1,2 *<br><br>-- | 1,3,4, 11,12, 14,17 | |
| X | DE - A - 1 503 433 (INTERNATIONAL BUSINESS MACHINES CORP.)<br> * Seite 2, Zeile 14 - Seite 4, Zeile 21; Seite 5, Zeile 10 - Seite 6, Zeile 17; Seite 8, Zeile 22 - Seite 10, Zeile 26; Abbildungen 1-3 *<br><br>-- | 1-4, 11,12, 16 | |
| X | US - A - 2 952 253 (H. SELIGMAN & S.H. DRAKE)<br> * Spalte 1, Zeilen 43-55; Spalte 2, Zeilen 33-54; Spalte 3, Zeile 72 - Spalte 4, Zeile 50; Abbildungen 1,4 *<br><br>-- | 1,2,4- 6,10, 11,13 | |
| | US - A - 3 489 172 (H.B. WHITMORE)<br> * Spalte 1, Zeile 11 - Spalte 3, Zeile 35; Abbildungen 1,2 *<br><br>-- | 1,2,5, 6,13, 17 | |
| | US - A - 2 979 067 (F. KERN, JR. & C. STANG JR./ MAXITROL COMPANY)<br> * Spalte 2, Zeile 35 - Spalte 3, Zeile 67; Abbildungen 1,2 *<br><br>---- | 2,3,5, 6,8,9 | |

RECHERCHIERTE SACHGEBIETE (Int. Cl.)

A 61 B 5/02
G 05 D 16/06

KATEGORIE DER GENANNTEN DOKUMENTE

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 27-05-1980 | RIEB |

EPA form 1503.1 06.78